# EUROPEAN PATENT SPECIFICATION

(11) **EP 3 252 143 B1**
(45) Date of publication and mention of the grant of the patent: **06.01.2021**
(21) Application number: 16172608.8
(22) Date of filing: 02.06.2016
(51) Int. Cl.: C12N 5/00, C12N 5/078

(54) **METHOD FOR PRODUCING A PLATELET-LYSATE-CONTAINING GEL**
VERFAHREN ZUR HERSTELLUNG EINES GELS MIT PLÄTTCHEN-LYSAT
PROCÉDÉ DE PRODUCTION D'UN GEL CONTENANT DU LYSAT PLAQUETTAIRE

(43) Date of publication of application: 06.12.2017
(73) Proprietor: PL BioScience GmbH, 52068 Aachen (DE)
(72) Inventor: Hemeda, Hatim, 52511 Geilenkirchen (DE); Wilkes, Christian, 48734 Reken (DE)
(74) Representative: Remus, Alvaro Johannes

(56) References cited:
- WO-A2-2013/076507
- US-A1- 2013 195 959
- GUDRUN WALENDA ET AL: "Human Platelet Lysate Gel Provides a Novel Three Dimensional-Matrix for Enhanced Culture Expansion of Mesenchymal Stromal Cells", TISSUE ENGINEERING. PART C, METHODS DEC 2008, vol. 18, no. 12, 1 December 2012 (2012-12-01), pages 924-934, XP55296430, US ISSN: 1937-3384, DOI: 10.1089/ten.tec.2011.0541
- Gudrun Walenda ET AL: "Human Platelet Lysate Gel Provides a Novel Three Dimensional-Matrix for Enhanced Culture Expansion of Mesenchymal Stromal Cells", TISSUE ENGINEERING. PART C, METHODS DEC 2008, vol. 18, no. 12, 1 December 2012 (2012-12-01), pages 924-934, XP055296430, US ISSN: 1937-3384, DOI: 10.1089/ten.tec.2011.0541

## Description

### Background of the invention

The invention relates to the use of a lyophilized platelet lysate material for preparing a three-dimensional, gel-like substrate for cultivating cells.

### Prior art

Cells are usually cultured in media that are supplemented with fetal calf serum (FCS) or other blood sera in order to provide the cells with essential growth factors. Although not any cell type actually needs serum for growth, most of the sensitive cell types, e.g., stem cells, are by now still dependent on serum-containing media. However, in light of therapeutic applications there is a growing interest to avoid the use of FCS due to potential xenogeneic immune reactions (e.g. anti-FCS antibodies), bovine pathogens (viruses and prions) and a high lotto-lot variability that hampers reproducibility of results (Heiskanen et al., 2007; Sundin et al., 2007). Chemically defined synthetic culture media are now commercially available, but their precise composition is often undisclosed and they often include other xenogeneic components such as recombinant growth factors.

Human platelet lysate (HPL) represents another attractive alternative to FCS. HPL can be generated from common platelet units by a simple freeze-thaw procedure. It is even possible to use HPL from the same donor to further minimize the risk of immunological side effects or viral infections. These studies were performed with mesenchymal stromal cells (MSC) from bone marrow and recently it has been demonstrated that HPL can also be used for isolation of MSC from human adipose tissue (Blande et al., 2009).

A method for preparing blood platelet lysate and a HPL medium is known from EP 0 413 727 B1. According to this known method citrate is added to whole blood in order to avoid coagulation. Plasma derived from this blood is centrifuged so as to obtain a platelet-rich paste and Ca²⁺ is then added for lysing the platelets and coagulating the remaining components. The filtered clear lysate is mixed with a conventional nutrient composition so as to obtain a HPL-containing medium.

Furthermore, WO 2008/034803 A1 discloses a cell culture medium supplement comprising plasma-free platelet lysate. The supplement further comprises additional substances such as albumin and dextran. In order to prepare the supplement, platelets are lysed by freezing and thawing them and acid citrate dextrose (ACD) or citrate phosphate dextrose (CPD) is added as anti-coagulant.

However, many cells, e.g., mesenchymal stromal cells (MSC), grow in a plastic adherent monolayer until they are contact inhibited. Sooner or later, the proliferation rate of the cells decays until they ultimately reach a senescent state. This is accompanied by enlarged morphology, reduced expression of surface markers and decreased differentiation potential. This process does not occur in parallel across cells in culture due to different subpopulations, apoptosis, and last but not least, contact inhibition within colonies. Lower seeding density facilitates higher intervals for cell passaging and hence, proliferation can only occur at the rim of the larger colonies. Consequently, when using plastic adherent monolayer cultures, it is hardly possible to accomplish a reliable expansion and to minimize contact inhibition. Moreover, cell passaging is not possible without using a cell-damaging peptidase treatment either. With stem cell cultures, it is another disadvantage of plastic adherent monolayer cultures that the plastic surface may cause undesired differentiation of the cells.

WO 2013/000672 A1 discloses a method for cultivating cells using a liquid medium comprising blood platelet lysate and a gelatinization-inhibiting substance. The cells are cultivated on the surface of and/or within a three-dimensional, gel-like substrate comprising blood platelet lysate but being substantially devoid of a gelatinization-inhibiting substance, wherein the liquid medium is layered on the gel-like substrate so as to establish a two-phase system. Mesenchymal stromal cells (MSCs) cultured on this gel reveal significantly higher proliferation rates in comparison to MSCs cultured on tissue culture plastic (TCP). However, although the gel-like substrate is well-suited to cultivate cells, it is still a drawback that the gel has to be prepared at the place of use as it cannot be transported over long distance and is not stable over a longer period of time.

US 2013/0195959 A1 discloses a method of preparing a composition suitable for therapeutic use or as a culture medium, comprising steps of concentrating platelets from a platelet source to form a platelet rich portion of the platelet source, and lysing the platelets in the platelet rich portion to form a plurality of lysates. An additional step includes lyophilizing the lysates to form lyophilized platelet lysates in a composition with released concentrations of available growth factors, cytokines, and chemokines.

Walenda et al. (2012) disclose a human platelet lysate (HPL) that can be used as a substitute for fetal calf serum without the risk of xenogeneic immune reactions or transmission of bovine pathogens. Heparin needs to be added as anticoagulant before addition of HPL to culture medium, otherwise, HPL-medium forms a gel within one hour. It is demonstrated that HPL-gels over-layered by a liquid HPL-medium, which - apart from heparin - consists of the same components as the HPL-medium, provide a suitable 3D-matrix for cell culture. Mesenchymal stromal cells (MSCs) grow in several layers at the interface between HPL-gel and HPL-medium without contact with any artificial biomaterials. The HPL-3D-matrix has many advantages over conventional plastic surfaces as it facilitates enhanced CFU-f outgrowth and enables increased proliferation rates, higher cell densities, and nonenzymatic passaging procedures for culture expansion of MSCs.

WO 2013/076507 A2 discloses a pharmaceutical composition comprising a platelet lysate which can be therapeutically applied to wounds, such as ulcerated wounds, anal fissures, vaginal atrophy or wrinkles. The composition or lysate is lyophilized to produce a stabilized, freeze-dried powder that contains growth factors and which can be used for therapeutic application in wound healing, but also other therapeutic applications in which there is an increased need for delivery of natural growth factors.

### Summary of the invention

It is the object of the invention to provide a use of a lyophilized platelet lysate material for preparing a three-dimensional, gel-like substrate for cultivating cells that can be easily transported and stored over a long period without significant impact on its cell culture properties.

The object is achieved by the use of a lyophilized dry platelet lysate material, in particular the platelet lysate material produced in the method as described herein, as a ready-to-use material for preparing a three-dimensional, gel-like substrate for cultivating cells, wherein the lyophilized platelet lysate material is free of any gelatinization-inhibiting substance. In an advantageous embodiment of the invention the cells may be, for example, mesenchymal stromal cells.

The method for producing the material for preparing a three-dimensional, gel-like substrate comprises:
a) Providing a thrombocyte concentrate;
b) Harvesting blood platelets from the thrombocyte concentrate;
c) Lysing the blood platelets so as to produce a platelet lysate, and
d) Lyophilization of said platelet lysate, so as to provide a dry platelet lysate material.

One step prior formation of a gel-like substrate includes lyophilizing the platelet lysate to form a dry, powder-like platelet lysate material. Surprisingly, the lyophilization step has no impact on the properties of the gel and provides a more stable supplement by increasing the shelf life for long time period under quality-controlled conditions. That is, the lyophilized platelet lysate material is stable and can be stored over a long period. Moreover, the lyophilized material can be easily transported so that the complete gel composition can be delivered to the place of use. The user does not need to accomplish the whole process of producing a platelet lysate gel at the place of use. Instead, the lyophilized platelet lysate material is produced under standardized and controlled conditions in a production facility and the lyophilized dry material is then delivered to the user as a kind of ready-to-use material which can be easily reconstituted by the user.

Gelatinization of pure human platelet lysate or a composition comprising human platelet lysate, which is free of any gelatinization-inhibiting substance such as heparin, occurs spontaneously at approximately 37°C within an hour. That is, addition of coagulation-inducing agents, such as thrombin and/or calcium gluconate, is not necessary for gelatinization. Accordingly, if the lyophilized platelet lysate material is reconstituted by the addition of water, the material spontaneously gelatinizes at the place of use so that a completely functional platelet lysate gel is produced.

At least one medium component can be added to the platelet lysate so as to adapt the platelet lysate gel to the specific needs of the cells to be cultivated. The medium component may comprise, for example, a standardized medium composition for cell culture, preferably Dulbecco's Modified Eagles medium (DMEM), in particular DMEM low glucose (DMEM-LG). Thus, a medium containing essential growth factors and all necessary nutrients for cell cultures without undesired serum components can be provided.

The platelet lysate gel material may comprise 1-50 % blood platelet lysate, preferably 1-40 % blood platelet lysate, more preferred 1-30 % blood platelet lysate, more preferred 1-20 % blood platelet lysate, more preferred 5-15 % blood platelet lysate, in particular approximately 10 % blood platelet lysate.

The blood platelets may be lysed by a freeze-thaw process. For example, the harvested platelet units may be frozen twice at -80°C and respectively re-thawed at 37°C.

For example, the platelet lysate gel material may essentially consist of 10 % HPL, 89 % DMEM, and 2mM L-Glutamine.

It turned out that the resulting gel-like substrate is a perfect substrate for expanding adherent cells, especially mesenchymal stromal cells (MSC) or fibroblasts. The use of a gel-like substrate comprising blood platelet lysate avoids the risk of xenogenic immunoreactions or transmission of bovine pathogens and is also more efficient than fetal calf serum (FCS) for large-scale expansion of cells, especially MSC from adipose tissue. For example, human platelet lysate (HPL) significantly increases the proliferation rate and maximal number of cumulative population doublings of adipose tissue derived MSC.

For example, the cells to be cultivated are stem or progenitor cells, particularly preferred mesenchymal stem cells, in particular mesenchymal stromal cells. In another exemplary embodiment of the invention, the cells are mesodermal cells, preferably fibroblasts or mesenchymal stromal cells. But, obviously, also other types of cells, for example, cells derived from ectodermal or endodermal lineages, or even primary cells, can be cultivated.

The term "lyophilization" as used herein refers to a "freeze-dry" process comprising the conversion of water from a frozen state to a gaseous state without going through a liquid state. The freeze-dry process removes moisture from a water-containing material while the material remains frozen. The basic process of lyophilization comprises the following steps: freezing, primary drying (sublimation), and secondary drying (desorption). At first, a dissolved and/or suspended substance is frozen at a low temperature (for example, -60 C°). Slow freezing produces larger crystals which allow the sublimating material to escape. Some products form a glassy material and annealing may be required during the freezing process. Annealing, first lowering the temperature then raising the temperature and then lowering it again, locks the constituents in place and then allows the crystals to grow. Freezing can range from 1 hour to 24 hours, depending on the application. In step 2 (primary drying) the water or diluent is then extracted via vacuum, resulting in a porous, dry "cake". Sublimation occurs under vacuum with the product temperature below its critical temperature. This is typically the longest process. At the end of the primary drying cycle, the product will have 3 to 5 % moisture content. There is a final drying step (secondary drying) to remove residual unfrozen water molecules. This is done by heating the product. Secondary drying can result in moisture levels of about 0.5 %.

The invention is further described in detail with reference to the figures.

### Brief description of the figures

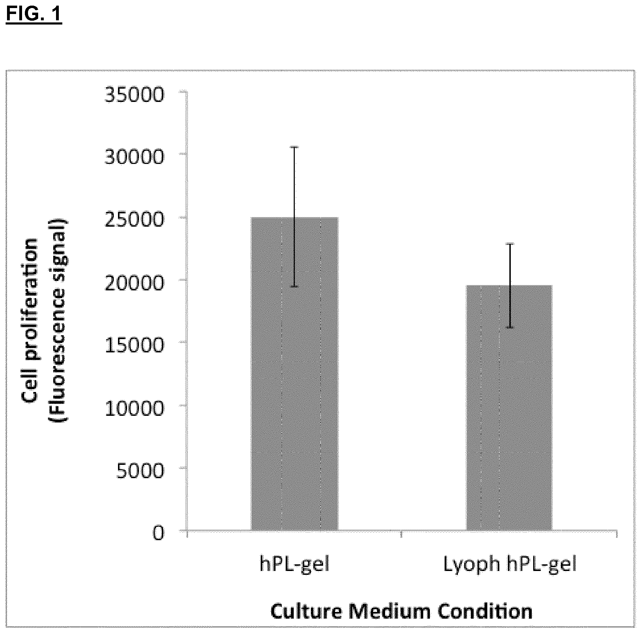

**Figure 1** shows a bar diagram showing the proliferation rate of MSCs on conventional hPL-gel and hPL-gel derived from a lyophilized dry PL material. Cells were grown either on normal hPL-gel or on a hPL-gel derived from a lyophilized dry PL material (Lyo-hPL-gel) for 15 days. Cell proliferation measurements revealed similar proliferation rates on both substrates.

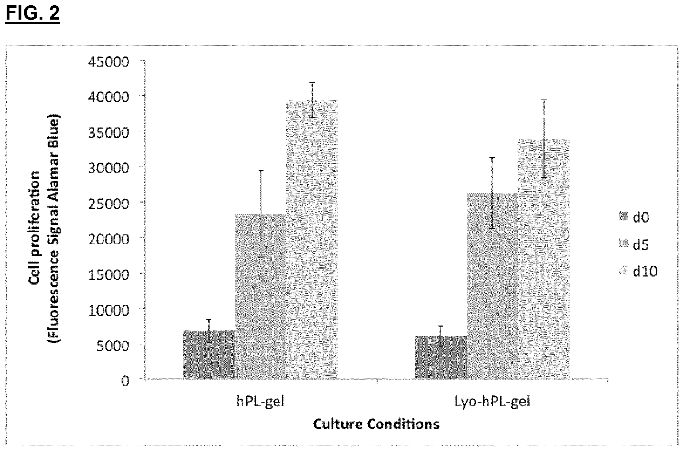

**Figure 2** shows a bar diagram showing the proliferation rate of MSCs after long-term cultivation on conventional hPL-gel and hPL-gel derived from a lyophilized dry PL material. Cells were grown either on normal hPL-gel or on hPL-gel derived from a lyophilized dry PL material (Lyo-hPL-gel) for 5 and 10 days. Cell proliferation measurements revealed a similar proliferation rates on both substrates.

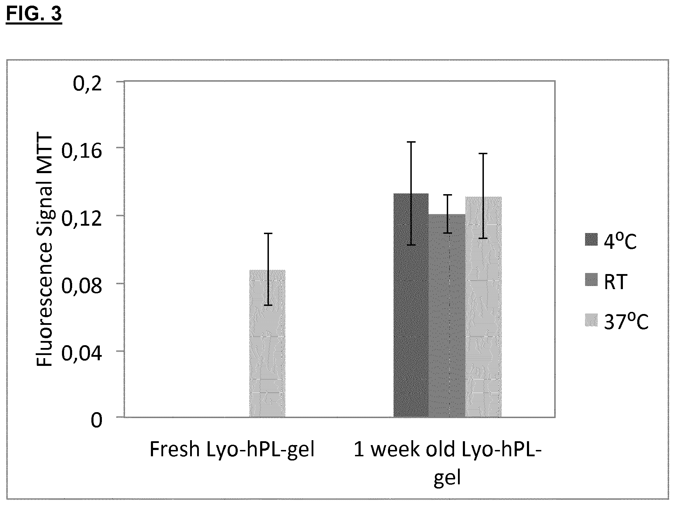

**Figure 3** shows a bar diagram showing the proliferation rate of MSCs grown on freshly prepared hPL-gel derived from a lyophilized dry PL material and one week old hPL-gel derived from a lyophilized dry PL material. Gel plates were prepared and stored at RT, 4°C and at 37°C. Cells were grown on hPL-gel derived from a lyophilized dry PL material (Lyo-hPL-gel) for 7 days.

### Description of exemplary and preferred embodiments of the invention

Mesenchymal stromal cells (MSCs) are a biologically important cell population that is able to support hematopoiesis, can differentiate along mesenchymal and nonmesenchymal lineages *in vitro,* is capable of suppressing alloresponses, and appears to be nonimmunogenic. These properties suggest emerging roles for mesenchymal stromal cells in cell therapy.

MSC were isolated from the caput femoris upon hip fracture after written consent using guidelines approved by the Ethic Committee of the University of Aachen. Bone fragments were flushed with PBS and mononuclear cells (MNC) subsequently isolated by a density gradient on Biocoll (Biochrom AG, Berlin, Germany). Cells were cultured in fibronectin coated cell culture dishes (Greiner, Kremsmunster, Austria) in Dulbecco's Modified Eagles Medium-Low Glucose (DMEM-LG; PAA, Pasching, Austria) with 2 mM L-glutamine (Sigma Aldrich, St. Louis, MO, USA) and 100 U/mL penicilline/streptomycine (pen/strep; Lonza, Basel, Switzerland). Culture medium was supplemented with FCS (HyClone, Bonn, Germany) or HPL as indicated. Culture medium was always changed twice per week. When reaching 80% confluence cells were trypsinized, counted by in a Neubauer counting chamber (Brand, Wertheim, Germany) and replated at 10⁴ cells/cm². Cumulative population doublings were calculated from the first passage onward.

Human platelet lysate (HPL) was generated from platelet units of healthy donors as described by Horn et al. (2010). In particular, HPL was generated by apheresis using the Trima Accel collection system (CaridianBCT, Garching, Germany). Such thrombocyte concentrates had a platelet content of 2.0 to 4.2×10¹¹ platelets in 200 mL plasma supplemented with Acid-Citrate-Dextrose (ACD) (1:11 v/v). Platelet units were aliquoted, twice frozen at -80°C, re-thawed at 37°C and centrifuged at 2600 *g* for 30 min at 4°C to remove cell fragments and pooled in equal amounts. The supernatant was filtered through 0.22 µm GD/X PVDF filter device (Whatman, Dassel, Germany), optionally supplemented with 2 U/mL heparin to avoid gelatinization, and stored at -80°C.

Cells were cultured in cell culture dishes (Greiner, Kremsmunster, Austria) in Dulbecco's Modified Eagles Medium-Low Glucose (DMEM-LG; PAA) with 2 mM L-glutamine (Sigma Aldrich, St. Louis, MO, USA) and 100 U/mL penicillin/streptomycin (pen/strep; Lonza, Basel, Switzerland). Culture medium/substrate was supplemented with HPL as indicated in the specification. Cells were cultured at 37°C in a humidified atmosphere containing 5% carbon dioxide with medium changes twice per week.

Cell proliferation was evaluated via the Thiazolyl Blue Tetrazolium Bromide (MTT) assay. MSC were seeded on a 96-well plate (3,000 cells/well) in culture medium/substrate supplemented with 0, 1, 5, 10, or 20% of HPL as indicated in the specification. After 7 days of culture, cells were washed with PBS (PAA) and incubated with 1 mM MTT (Sigma-Aldrich) in PBS for 3.5 h. The excess solution was discarded and crystals were resolved in 4 mM HCI in isopropanol (both from Roth, Karlsruhe, Germany). Optical density (OD) was measured at wave length 590 nm with a Tecan Infinite 2000 plate reader (Tecan Trading, Mannedorf, Switzerland).

### Example

### Generation of human platelet lysate:

Platelet units were kindly provided by the Institute for Transfusion Medicine, RWTH Aachen University Medical School after their expiry date (5 days after harvesting). Platelet units were generated by apheresis using the Trima Accel Collection System (CaridianBCT, Garching, Germany) and comprise 2.0-4.2 × 10¹¹ platelets in 200 mL plasma supplemented with Acid-Citrate-Dextrose (ACD) (1:11 v/v). Aliquots of 45 mL were twice frozen at -80°C and re-thawed at 37°C and then centrifuged at 2,600 × *g* for 30 minutes. The supernatant was filtered through 0.2 µm GD/X PVDF filters (Whatman, Dassel, Germany) and stored at -80°C until use. Experiments of this study were performed with three HPL-pools each consisting of lysates of four donors but similar results were also observed using individual donor derived HPLs. HPLs consisted predominately of blood type A but a systematic analysis did not reveal any impact of the blood group.

### Culture medium and HPL-gel:

Culture medium consisted of Dulbecco's Modified Eagles Medium-Low Glucose (DMEM-LG; PAA, Pasching, Austria) with 2 mM L-glutamine (Sigma Aldrich, St. Louis, MO, USA) and 100 U/mL penicillin/streptomycin (pen/strep; Lonza, Basel, Switzerland) and HPL (10% if not indicated otherwise). Cells were cultured on tissue culture plastic (TCP; Greiner, Kremsmünster, Austria). For gel preparation, HPL containing cell culture medium without heparin was filled in cell culture plates. Due to the calcium comprised in the medium the coagulation cascade was initiated and the HPL-medium gelatinized within one hour at 37°C. That is, the blood platelet lysate, either pure or diluted, spontaneously gelatinizes at approximately 37°C without the necessity to add any coagulation-inducing agent such as, for example, thrombin and/or calcium gluconate. The resulting HPL-gel was used as cell culture substrate which was seeded with the same amount of MNC. For comparison, gels consisting of 80% collagen G (3 mg/mL collagen type I/III in 12 mM HCl; Biochrome, Berlin, Germany) were used as described before. Cells were cultured at 37°C in 6 well plates or T75 tissue culture flasks (Nunc Thermo Fisher Scientific, Langenselbold, Germany).

### Elasticity measurements of HPL gels:

For elasticity measurements, HPL-gels were formed in 5%, 10% and 20% concentrations using plastic cylinders (14 mm in diameter and 2 mm in height) as mold on top of thin cover slides. After gelatinization as described above, mold cylinders were removed and HPL-gels were overlaid with a cover slide of known mass. Gel thickness was determined using a confocal laser scanning microscope (LSM 510 Meta, Zeiss, Germany) equipped with a long distance 40x Achroplan (NA 0.6, PH 2) objective. Placement of additional cover slides of known weight and subsequent thickness measurement were repeated at least two times for each HPL-gel cylinder. From the measured quantities of gel thickness (h), total mass of cover slide(s) (m), thickness reduction due to loading (Dh), and gel cross-section (A), we calculated the elastic modules of the gel according to E= (m^{∗}g)/A^{∗}(h/□l) where g denotes the acceleration due to gravitation.

### Isolation of human mesenchymal stromal cells from bone marrow:

MSC were isolated from mononuclear cells (MNC) by plastic adherence. In brief, bone fragments from the *caput femoris* of patients undergoing femoral head prosthesis were flushed with phosphate-buffered saline (PBS). Subsequently, MNC were isolated by Ficoll density gradient centrifugation (Biocoll Separating Solution, density 1.077g/l, Biochrom AG, Berlin, Germany) and washed twice with PBS. At least 5 × 10⁵ MNC were then resuspended in culture medium either on TCP, HPL-gel or collagen-gels in 6-well plates. Cells were cultured at 37°C in a humidified atmosphere with 5% CO₂. The first medium exchange was performed after 48 hours to remove non-adherent cells. Thereafter, half-medium changes were performed twice per week and MSC were further passaged as described below.

### Isolation of human dermal fibroblasts:

Fibroblasts were isolated from skin samples of patients undergoing cosmetic surgery. Initially the cells were expanded in DMEM-LG supplemented with glutamine (PAA), penicillin/streptomycin (PAA) and 10% FCS (Biochrom, Berlin, Germany). Cells at passage 3 to 5 were then transferred to HPL-culture conditions as described above.

### Scanning electron microscopy:

Human MSC/HPL-gel matrix were fixed in 3% glutaraldehyde for at least 1 hour at room temperature, rinsed with PBS and dehydrated by incubating consecutively in 30%, 50%, 70%, 90%, and 100% ethanol for 10 minutes at room temperature. HPL-gels were then critical point dried in liquid CO₂ and sputter-coated with a 30 nm gold layer using an ion sputter coater (LEICA EM SCD 500). Samples were analyzed using an environmental scanning electron microscope at the electron microscope facility, RWTH Aachen University (ESEM XL 30 FEG, FEI, Philips, Eindhoven, Netherlands).

### Histo sections-HE staining:

For histological analysis, HPL-gels with cultured cells at the surface were fixed in 3.7% formaldehyde for 24 hours. The constructs were paraffin embedded, orientated on the perpendicular plane, cut with a rotating microtome at 2 µm thickness (Leica, Wetzlar, Germany) and stained with hematoxilin and eosin according to routine histology protocols.

### Proliferation assay and vitality analysis:

Cell proliferation was assessed via the methyl thiazolyl tetrazolium (MTT) assay. Briefly, MSC of passage 3-6 were seeded in 96-well plates (3,000 cells/cm²) on HPL-gels in culture medium supplemented with 1%, 5%, 10% or 20% HPL. After 7 days, cells were washed with PBS and incubated with 1 mM MTT (Sigma Aldrich, St. Louis, MO, USA) for 3.5 hours at 37°C. The excess solution was discarded and formazan crystals were resolved in 4 mM HCI in isopropanol (both from Roth, Karlsruhe, Germany). The absorbance was measured at 590 nm using a Tecan Infinite 200 plate reader (Tecan Trading, Switzerland). Each measurement included four technical replicas. Alternativly, proliferation was estimated by carboxyfluorescein diacetate N-succinimidyl ester (CFSE) staining as described before. Furthermore, nuclear staining or live dead staining was performed with DAPI (4',6-Diamidin-2-phenylindol; Molecular Probes) or with 5 µg/mL fluorescein diacetate (Sigma) and 0.5 µg/mL Propidium iodide (BD Pharmingen) respectively, after 7 days of culture on TCP or HPL-gels and analyzed with a Leica DM IL HC fluorescence microscope.

### Fibroblastoid colony-forming unit (CFU-f) assay:

The CFU-f assay was performed by plating MNC in a limiting dilution series in 96-well plates (330,000; 100,000; 33,000; 10,000; 3,000; and 1,000 cells per well; at least 24 wells per dilution step in each experiment) in parallel on both TCP and HPL-gel. Medium was exchanged after 48 hours for the first time and then twice per week. After 14 days in culture, cells were washed twice with PBS and labeled with MTT. All wells with cell growth scored positive and the CFU-f frequency was determined by Poisson statistics (L-calc; Stem Cell Technologies; Vancouver, Canada).

### Passaging and long-term culture of MSC:

MSC on TCP were continuously passaged after reaching a confluency level of 80% using trypsin-EDTA solution 0.25 % (Sigma Aldrich, St. Louis, MO, USA). Cells on HPL-gel were harvested together with the gel by pipetting. Thereby, the gel-MSC mix was diluted with culture medium and then plated onto fresh HPL-gel coated well plates. To determine the MSC cell number, a fraction of the MSC/HPL-gel mixture was seeded on TCP for 24 hours to facilitate attachment to the plastic surface. The plastic-adherent cells were then trypsinized and the cell number was finally determined using Neubauer counting chamber (Brand, Wertheim, Germany). Cell population doublings per passage (PDP) and cumulative population doublings (cPD) were calculated as known in the art. Alternatively, MSC/HPL-gel was incubated with 20 µg plasmin (stock solution 2.76 mg/mL; Enzyme Research Laboratories, Indiana, USA) for 18 hours at 37°C.

MSCs on HPL-gel were harvested by pipetting with or without additional incubation with trypsin/EDTA for 5 minutes, or with plasmin as described above. All cells were reseeded on TCP and after 1 day the number of adherent and non-adherent cells was estimated using the MTT assay.

### Immunophenotypic analysis:

For characterization of MSC we have used a panel of surface markers. MSC were isolated and cultured for three passages either on TCP or on HPL-gel. To remove the HPL-gel prior to flowcytometric analysis we have either seeded the cells for one additional passage on TCP or we have harvested the HPL-gel by pipetting and washed the cells once in culture medium. Each cell preparation was stained in parallel with the following monoclonal antibodies: mouse anti-human CD14-APC (clone M5E2), CD29-PE (clone MAR4), CD31-PE (clone WM59), CD34-APC (clone 8G12), CD45-APC (clone HI30), CD73-PE (clone AD2), CD90-APC (clone 5E10) (all BD Bioscience, Heidelberg, Germany) and CD105-FITC (clone MAR-226; ImmunoTools, Friesoythe, Germany). Analysis was performed using a FACS Canto II cytometer (BD Bioscience, Heidelberg, Germany) and the collected data were analyzed with WinMDI 2.9 software (Windows Multiple Document Interface for Flow Cytometry). For statistical comparison of different measurements we have normalized the fluorescence intensities to the corresponding autofluorescence measurements.

### In vitro differentiation:

Freshly isolated MSC were directly seeded on either HPL-gels or TCP and expanded for three passages prior to *in vitro* differentiation. Differentiation towards osteogenic, adipogenic and chondrogenic lineage was then induced as known in the art. This differentiation of HPL-gel-MSC was either performed on HPL-gel or upon transfer to TCP. Chondrogenic differentiation was performed in 3D pellet culture upon centrifugation at 400 × g for 7 min. After three weeks, differentiation was analyzed by Alizarin Red stain (osteogenic differentiation), with the green fluorescent dye BODIPY (4,4-difluoro-1,2,5,7,8-pentamethyl-4-bora-3a,4a-diaza-s-indacene; Molecular Probes, Eugene, USA; adipogenic differentiation), or with Alcian blue and nuclear fast red (for sections of chondrogenic differentiated samples) according to routine histology protocols and analyzed with a Leica DM IL HC fluorescence microscope (Leica, Wetzlar, Germany). Furthermore, adipogenic differentiation was quantified on TCP and HPL-gel by counterstaining of all cells with DAPI and direct counting of non-differentiated in relation to differentiated cells (at least 300 cells per experiment).

### Statistics:

Results are expressed as mean ± standard deviation of independent experiments. To estimate the probability of differences we have adopted the paired two-sided Student's T-test.

### Lyophilization:

Freezing is done by placing the gel in a freeze-drying flask and rotating the flask in a shell freezer which is cooled by mechanical refrigeration, dry ice and methanol, or liquid nitrogen. On a larger scale, freezing can be accomplished using a freeze-drying machine. In this step, it is important to cool the material below its triple point, the lowest temperature at which the solid and liquid phases of the material can coexist. This ensures that sublimation rather than melting will occur in the following steps. Larger crystals are easier to freeze-dry. To produce larger crystals, the product should be frozen slowly or can be cycled up and down in temperature (annealing). The freezing temperatures are between -50 C and -80 C.

During the primary drying phase, the pressure is lowered (to the range of a few millibars), and enough heat is supplied to the frozen gel for the ice to sublime. In this initial drying phase, about 95% of the water in the material is sublimated. This phase may be slow because, if too much heat is added, the material's structure could be altered. In this phase, pressure is controlled through the application of partial vacuum. The vacuum speeds up the sublimation, making it useful as a deliberate drying process. Furthermore, a cold condenser chamber and/or condenser plates provide a surface for the water vapour to re-solidify on. Condenser temperatures are typically below -50 C (-60 F).

The secondary drying phase aims to remove unfrozen water molecules, since the ice was already removed in the primary drying phase. In this phase, the temperature is raised higher than in the primary drying phase, and can even be above 0 °C, to break any physico-chemical interactions that have formed between the water molecules and the frozen material. The pressure can also be lowered in this stage to encourage desorption (for example, in the range of microbars, or fractions of a pascal).

After the freeze-drying process is complete, the vacuum is broken with an inert gas, such as nitrogen, before the material is sealed. At the end of the operation, the final residual water content in the product is extremely low, around 1% to 4%.

### Results:

Cells grown on the hPL-gel derived from a lyophilized dry PL material show similar cell proliferation and expansion rates and no effect of the lyophilization on the differentiation potential and the immunophenotype profile of seeded MSCs compared to the fresh prepared hPL-gel without lyophilization can be observed **(****Figure 1****).** Using the hPL-gel derived from a lyophilized dry PL material, long-term cultivation of MSCs revealed the same results without any differences in cell growth properties **(****Figure 2****).** Cell proliferation measurements revealed a stable gel under different storage conditions. In particular, a hPL-gel derived from a lyophilized dry PL material that was stored for one week at room temperature (RT), 4°C or 37°C is still stable and cell proliferation even seems to be increased using such gel **(****Figure 3****).** In total, this method facilitates transportation of the gel material and ensures a higher shelf life of the platelet lysate gel without any impact on cell culture properties.

For example, the method according to the invention comprises the following steps. At first, human blood platelet units are lysed by a freeze-thaw process. After centrifugation and filtration, the platelet lysate is collected. The platelet lysate is then dried by lyophilization so as to produce a dry human platelet lysate material (Lyo-hPL-gel). The lyophilized dry PL material can be easily reconstituted by the addition of water at the place of use in order to provide a completely functional platelet lysate-gel (hPL-gel) which can be used for cultivating living cells without any limitations. Gelatinization of the platelet lysate, which is free of any gelatinization-inhibiting substance such as heparin, occurs spontaneously at approximately 37°C, resulting in the platelet lysate gel. Accordingly, the blood platelet lysate is allowed to gelatinize at approximately 37 °C so as to obtain a human platelet lysate gel (hPL-gel). In order to modify the hPL-gel, a suitable cell culture medium and/or suitable supplements may be added to the gel material after the Lyo-hPL-gel was dissolved with water.

### Non-patent literature

Blande, I. S.; Bassaneze, V.; Lavini-Ramos, C.; Fae, K. C.; Kalil, J.; Miyakawa, A. A.; Schettert, I. T.; Krieger, J. E.: Adipose tissue mesenchymal stem cell expansion in animal serum-free medium supplemented with autologous human platelet lysate. Transfusion 49: 2680-2685; 2009.
Heiskanen, A.; Satomaa, T.; Tiitinen, S.; Laitinen, A.; Mannelin, S.; Impola, U.; Mikkola, M.; Olsson, C.; Miller-Podraza, H.; Blomqvist, M.; Olonen, A.; Salo, H.; Lehenkari, P.; Tuuri, T.; Otonkoski, T.; Natunen, J.; Saarinen, J.; Laine, J.: N-glycolylneuraminic acid xenoantigen contamination of human embryonic and mesenchymal stem cells is substantially reversible. Stem Cells 25:197-202; 2007.
Horn, P.; Bokermann, G.; Cholewa, D.; Bork, S.; Walenda, T.; Koch, C.; Drescher, W.; Hutschenreuther, G.; Zenke, M.; Ho, A.; Wagner, W.: Comparison of Individual Platelet Lysates for Isolation of Human Mesenchymal Stromal Cells. Cytotherapy 12: 888-898; 2010.
Sundin, M.; Ringden, O.; Sundberg, B.; Nava, S.; Gotherstrom, C.; Le Blanc K.: No alloantibodies against mesenchymal stromal cells, but presence of anti-fetal calf serum antibodies, after transplantation in allogeneic hematopoietic stem cell recipients. Haematologica 92:1208-1215; 2007.
Walenda, G.; Hemeda, H.; Schneider, R.K.; Merkel, R.; Hoffmann, B.; Wagner, W.: Human platelet lysate gel provides a novel three dimensional-matrix for enhanced culture expansion of mesenchymal stromal cells. Tissue Engineering Part C, Volume 18, No. 12, 924-934; 2012.

## Claims

1. Use of a lyophilized dry platelet lysate material as a ready-to-use material for preparing a three-dimensional, gel-like substrate for cultivating cells, wherein the lyophilized platelet lysate material is free of any gelatinization-inhibiting substance.

2. Use according to claim 1, wherein the cells are mesenchymal stromal cells.

## Patentansprüche

1. Verwendung eines lyophilisierten trockenen Plättchenlysat-Materials als ein gebrauchsfertiges Material zur Herstellung eines dreidimensionalen gelartigen Substrats zur Kultivierung von Zellen, wobei das lyophilisierte Plättchenlysat-Material frei von jeglicher Gelierung-hemmenden Substanz ist.

2. Verwendung nach Anspruch 1, wobei die Zellen mesenchymale Bindegewebszellen sind.

## Revendications

1. Utilisation d'un matériau de lysat de plaquettes sec lyophilisé comme matériau prêt à l'emploi pour la préparation d'un substrat tridimensionnel de type gel pour la culture de cellules, dans lequel le matériau de lysat de plaquettes lyophilisé est exempt de toute substance inhibant la gélatinisation.

2. Utilisation selon la revendication 1, dans laquelle les cellules sont des cellules stromales mésenchymateuses.
